# EUROPEAN PATENT APPLICATION

(11) **EP 4 183 770 A1**
(43) Date of publication of application: **24.05.2023**
(21) Application number: 21209660.6
(22) Date of filing: 22.11.2021
(51) Int. Cl.: C07C 219/06

(54) **CONTINUOUS FLOW PROCESS FOR PRODUCTION OF CATIONIC LIPIDS**

(71) Applicant: Max-Planck-Gesellschaft zur Förderung der Wissenschaften e.V., 80539 München (DE)
(72) Inventor: SEEBERGER, Peter, 14532 Kleinmachnow (DE)
(74) Representative: Arth, Hans-Lothar

(57) **Abstract**

The present invention is directed to a method for continuous production of cationic lipids. Said cationic lipids are particularly useful in combination with other lipid components for forming lipid nanoparticles with oligonucleotides (e.g. mRNA) to facilitate delivery of therapeutically active nucleic acids.

## Description

The present invention is directed to a method for continuous production of cationic lipids. Said cationic lipids are particularly useful in combination with other lipid components for forming lipid nanoparticles with oligonucleotides (e.g. mRNA) to facilitate delivery of therapeutically active nucleic acids.

### Background of the invention

Although nucleic acid vaccines, such as mRNA vaccines have been under development for more than two decades, only recently mRNA vaccines have been propelled in the focus of the biotechnology and pharmaceutical industry due to the COVID-19 pandemic.

Free RNA is susceptible to nuclease digestion in plasma and has limited ability to gain access to the intracellular compartment, where the relevant translation machinery resides. These drawbacks have been overcome recently due to the development of lipid nanoparticle delivery systems that block degradation of RNA in plasma and facilitate cellular uptake. It has been found that lipid nanoparticle systems made of cationic lipids with other lipid components, such as neutral lipids, cholesterol, PEG, or PEGylated lipids, have been particularly efficient.

The cationic lipid ALC-0315 having four alkyl tails and an ionizable tertiary amine group has recently be identified as one lipid component of BioNTech's approved BNT162b2 vaccine (Buschmann et al. Vaccines 2021, 9, 65).

Some synthetic methods of cationic lipids, such as ALC-0315 are known, but suffer from low reaction yields and require many purification steps which render these processes laborious. WO 2016/176330 A1 discloses a batch process starting from an acid chloride and a diol (e.g.2-hexyldecanoyl chloride and 1,6-hexanediol for ALC-0315 synthesis). At first, the acid chloride and the diol are esterified, which required 2 equivalents of triethylamine to neutralize the formed hydrogen chloride. After purification, the obtained alcohol is oxidized to the corresponding aldehyde with pyridinium chlorochromate and subsequently, the purified aldehyde is subjected to reductive amination with sodium triacetoxy borohydride and acetic acid in the presence of a primary amine (e.g. 4-amino-1-butanol). In case of ALC-0315 synthesis, an overall reaction yield of 6% was reported.

A similar batch process is disclosed in WO 2017/075531 A1, wherein an activated carboxylic acid is utilized instead of an acid chloride. An overall reaction yield of 13% was reported.

In view of the ongoing COVID-19 pandemic there is an increasing demand for mRNA technology-based vaccines, which utilize lipid nanoparticle systems. As already mentioned above, the synthetic methods for cationic lipids known in the art suffer from low reaction yields and are performed with batch methods. Batch methods have disadvantages, such as scale-up, high cost and elaborate multiple purification steps.

Moreover, the methods from the prior art require stoichiometric high molecular weight reagents, such as dicyclohexylcarbodiimide and 4-dimethylaminopyridine that in turn, produce high molecular weight stoichiometric organic byproducts thereby resulting in low atom economy. In addition, toxic (genotoxic and carcinogenic) reagents such as pyridinium chlorochromate are used in the methods described in the literature.

Thus, there is a need for efficient synthesis of the lipid components to ensure the supply of sufficient amount of vaccine.

Therefore, the objective of the present invention is to provide a more efficient method for the synthesis of cationic lipids of general formula (I).

The objective of the present invention is solved by the teaching of the independent claims. Further advantageous features, aspects and details of the invention are evident from the dependent claims, the description, the figures, and the examples of the present application.

### Description of the invention

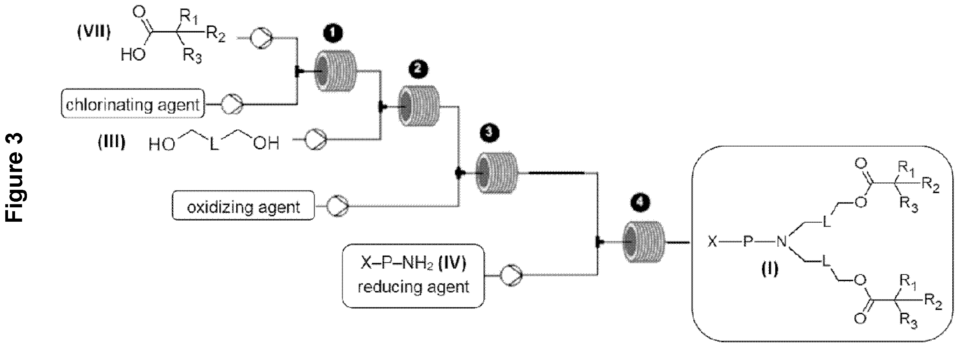

It was found that a cationic lipid of general formula (I) can be prepared in a continuous flow process from an acid chloride of formula (II) and a diol compound of formula (III) as depicted in Figure 3, wherein the esterification of the acid chloride of formula (II) and the diol compound of formula **(III)** is performed in the absence of a base.

The continuous flow process according to the present invention can be readily scaled up by a simple continuous flow system comprising four continuous flow reactors and can be performed in a fully continuous manner without solvent exchanges, or interruptions for intermediate workups or purifications. Besides the easier scalability, the continuous flow process allows a better control of the reaction parameters, higher reproducibility of product yields and purities as well as higher degree of automation and real-time process control.

Thus, the present invention is directed to a process for producing a compound of general formula (I) in a continuous manner or in other words the present invention is directed to a continuous flow process for producing a compound of general formula (I): 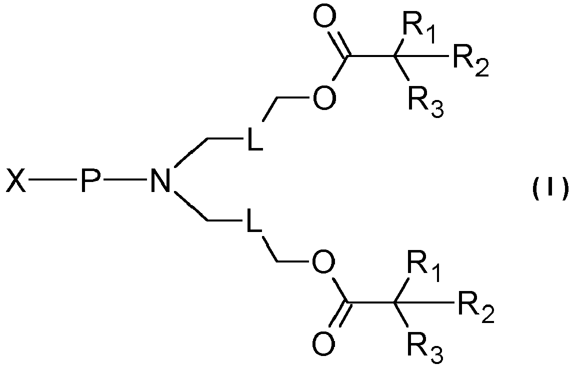 wherein R₁, R₂ and R₃ independently from each other represent -H, C₁₋₂₄ alkyl , or C₂₋₂₄ alkenyl;
L represents C₁₋₁₂ alkylene, C₂₋₁₂ alkenylene, C₂₋₁₂ alkynylene, C₃₋₈ cycloalkylene, C₃₋₈ cycloalkenylene, or -(CH₂-O-CH₂)₁₋₆;
P represents C₁₋₂₄ alkylene, C₂₋₂₄ alkenylene, C₂₋₂₄ alkynylene, or -(CH₂)ₘ-(CH₂-O-CH₂)₁₋₆-(CH₂)ₙ-;
X is selected from -H, -OR₄, -CN, -C(O)OR₄, -OC(O)OR₄, and -NR₄R₅;
R₄ and R₅ independently from each other are selected from -H or C₁₋₁₂ alkyl;
wherein m and n are integers independently selected from 0 and 1;
the process comprising the following steps:
   A) providing a continuous flow of a solution comprising acid chloride (II) and diol compound **(III);** 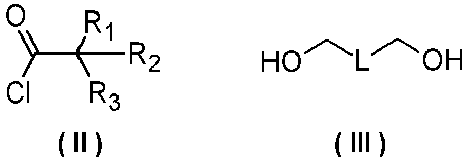
   B) continuously producing a compound of formula **(IV)** from the continuous flow of the solution comprising acid chloride **(II)** and diol compound **(III)** in a continuous flow reactor in the absence of a base; 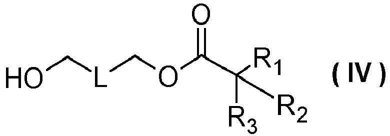
   C) combining the continuous flow of a solution containing the compound of formula **(IV)** with a continuous flow of a mixture comprising an oxidizing agent;
   D) continuously producing a compound of formula **(V)** from the combined continuous flow obtained in step C) in a continuous flow reactor; 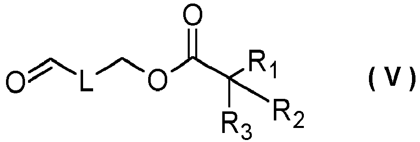
   E) combining the continuous flow containing the compound of formula **(V)** obtained in step D) with a continuous flow of a solution comprising a reducing agent and with a continuous flow of a solution comprising an amine compound of formula **(VI);** and

      X-P-NH₂ **(VI)**
   F) continuously producing the compound of formula **(I)** from the combined continuous flow obtained in step E) in a continuous flow reactor.

The continuous flow of a solution comprising acid chloride **(II)** and diol compound **(III)** of step A) may be obtained by combining a continuous flow of a solution comprising the acid chloride **(II)** and a continuous flow of a solution comprising the diol compound **(III).** Thus, the present invention is directed to a continuous flow process for producing a compound of general formula **(I)** comprising the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor,
wherein the continuous flow of a solution comprising acid chloride **(II)** and the diol compound **(III)** of step A) is obtained by combining a continuous flow of a solution comprising the acid chloride **(II)** and a continuous flow of a solution comprising the diol compound **(III).**

The continuous flow of a solution comprising the acid chloride **(II)** may be obtained by reacting a carboxylic acid of formula **(VII)** with a chlorinating agent. Suitable chlorinating agents include, but are not restricted to, thionyl chloride, oxalyl chloride, bis(trichloromethyl)carbonate or phosgene, 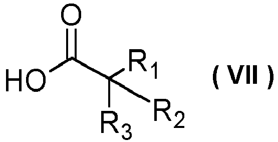 wherein R₁, R₂ and R₃ have the meanings as defined herein.

Thus, the present invention is also directed to a continuous flow process for producing a compound of general formula **(I)** comprising steps A1) and A2) instead of step A):
A1) providing a continuous flow of a solution comprising a carboxylic acid of formula **(VII)** and a chlorinating agent;
A2) continuously producing an acid chloride of formula **(IV)** from the continuous flow of the solution comprising the carboxylic acid of formula **(VII)** and the chlorinating agent in a continuous flow reactor;
B) continuously producing a compound of formula **(IV)** from the continuous flow of the solution comprising acid chloride **(II)** and diol compound **(III)** in a continuous flow reactor;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor,
wherein the continuous flow of a solution comprising acid chloride **(II)** and the diol compound **(III)** of step A) is obtained by combining a continuous flow of a solution comprising the acid chloride **(II)** and a continuous flow of a solution comprising the diol compound **(III).**

Preferably, the chlorinating agent is selected from thionyl chloride, oxalyl chloride, bis(trichloromethyl)carbonate or phosgene. Most preferably, the chlorinating agent is thionyl chloride. Preferably, step A2) is performed at a reaction temperature between 23°C and 100°C, more preferably at a reaction temperature between 40°C and 100°C, even more preferably at a reaction temperature between 60°C and 90°C and most preferably at a reaction temperature of about 80°C. Most preferably, the chlorinating agent is thionyl chloride and step A2) is performed at a reaction temperature of about 80°C.

In one embodiment, preferably 0.005 molar equivalents to 0.2 molar equivalents of N,N-dimethylformamide, more preferably 0.007 molar equivalents to 0.15 molar equivalents of *N*,*N*-dimethylformamide, more preferably 0.01 molar equivalents to 0.1 molar equivalents of *N*,*N*-dimethylformamide, and most preferably 0.05 molar equivalents of N,N-dimethylformamide are added in step A2) in order to increase the reaction rate.

The inventors have found that the esterification reaction of step B) can be run in a significantly shorter amount of time, i.e. at a higher flow rate or at a shorter residence time of about 4 minutes, when the reaction is performed at elevated temperatures.

In a further embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor at a temperature between 40 °C and 150 °C in the absence of a base;
C) combining the continuous flow of a solution containing the compound of formula **(IV)** with a continuous flow of a mixture comprising an oxidizing agent;
D) continuously producing a compound of formula **(V)** from the combined biphasic continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor.

The inventors have surprisingly found that a cationic lipid of general formula **(I)** can be prepared in a continuous flow process by performing the esterification reaction of acid chloride of formula **(II)** and diol compound of formula **(III)** in step B) without the addition of a base. It was observed that neutralizing the two equivalents of hydrogen chloride formed during the esterification reaction leads to a heterogeneous reaction mixture due to precipitation of insoluble or weakly soluble salt. The precipitated salt may clog the lines of the continuous flow system and is therefore disadvantageous for the continuous flow process. The precipitation of amine salt occurs particularly in a continuous flow process due to the high concentrations.

A "base" as used herein refers to a compound that can be used for neutralizing the two equivalents of hydrogen chloride formed during the esterification reaction, such as amines (e.g. triethylamine, *N*,*N*-diisopropylethylamine, 4-dimethylaminopyridine, etc.), ammonia, basic metal salts like oxides, hydroxides, carbonates, or bicarbonates. It is to be understood that "without addition of a base" and "in the absence of a base" is synonymously used herein and refer to the esterification reaction in step B) without the presence of a base, neither by addition during the esterification reaction, nor by a basic continuous flow of a solution comprising acid chloride **(II)** and diol compound **(III).**

A "neutralization reaction" as used herein refers to a procedure for adjusting the pH of a reaction mixture, formed in the methods described herein, to a value of about 7 by using acid(s), base(s), aqueous alkaline solution(s) and/or aqueous acidic solution(s) An exemplarily neutralization reaction is the treatment of the acidic the continuous flow of a solution containing the compound of formula **(IV)** obtained from step B) with a base.

Moreover, the inventors could demonstrate that neutralizing the formed hydrogen chloride has a detrimental effect on the esterification of step B), such that lower reaction yields and selectivities in regard to the alcohol of formula **(IV)** were observed. Thus, the inventors could show that it is beneficial to remove the hydrogen chloride at a later stage of the continuous flow process according to the invention.

Therefore, the present invention is directed to a continuous flow process for producing a compound of general formula **(I)** comprising the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor;
C) combining the continuous flow of a solution containing the compound of formula **(IV)** with a continuous flow of a mixture comprising an oxidizing agent and a base;
C') neutralizing the continuous flow obtained in step C);
D) continuously producing a compound of formula **(V)** from the combined continuous flow obtained in step C') in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor.

In other words, the present invention is directed to a continuous flow process for producing a compound of general formula **(I)** comprising the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor, thereby forming hydrogen chloride;
C) combining the continuous flow of a solution containing the compound of formula **(IV)** with a continuous flow of a mixture comprising an oxidizing agent and a base;
C') neutralizing the hydrogen chloride formed in step B);
D) continuously producing a compound of formula **(V)** from the combined continuous flow obtained in step C') in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor.

In a particularly preferred embodiment, the neutralization reaction is performed after step B) and before step C) by steps B1'), B2') and B3'):
B1') combining the continuous flow of a solution containing the compound of formula **(IV)** with a continuous flow comprising an aqueous base,
B2') continuously performing a neutralization reaction in a continuous flow reactor at a reaction temperature below 23°C, and
B3') purifying compound of formula **(IV)** under continuous flow conditions.

Thus, the present invention is also directed to a continuous flow process for producing a compound of general formula **(I)** comprising the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
B1') combining the continuous flow of a solution containing the compound of formula **(IV)** with a continuous flow comprising an aqueous base,
B2') continuously performing a neutralization reaction in a continuous flow reactor at a reaction temperature below 23°C,
B3') purifying compound of formula **(IV)** under continuous flow conditions,
C) combining the continuous flow of a solution containing the compound of formula **(IV)** with a continuous flow of a mixture comprising an oxidizing agent;
D) continuously producing a compound of formula **(V)** from the combined biphasic continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor.

Preferably, the aqueous base is an aqueous solution of an alkali metal hydroxide. Preferably, the aqueous base is a 1 molar aqueous solution of an alkali metal hydroxide. Preferably, the aqueous base is an aqueous solution of lithium hydroxide, sodium hydroxide or potassium hydroxide. Most preferably, the aqueous base is a 1 molar aqueous solution of sodium hydroxide. In a preferred embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
B1') combining the continuous flow of a solution containing the compound of formula **(IV)** with a continuous flow comprising an aqueous sodium hydroxide solution,
B2') continuously performing a neutralization reaction in a continuous flow reactor at a reaction temperature below 23°C,
B3') purifying compound of formula **(IV)** under continuous flow conditions,
C) combining the continuous flow of a solution containing the compound of formula **(IV)** with a continuous flow of a mixture comprising an oxidizing agent;
D) continuously producing a compound of formula **(V)** from the combined biphasic continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor.

In a further preferred embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
B1') combining the continuous flow of a solution containing the compound of formula **(IV)** with a continuous flow comprising an aqueous base,
B2') continuously performing a neutralization reaction in a continuous flow reactor at a reaction temperature of 0°C,
B3') purifying compound of formula **(IV)** under continuous flow conditions,
C) combining the continuous flow of a solution containing the compound of formula **(IV)** with a continuous flow of a mixture comprising an oxidizing agent;
D) continuously producing a compound of formula **(V)** from the combined biphasic continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor.

In a further preferred embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
B1') combining the continuous flow of a solution containing the compound of formula **(IV)** with a continuous flow comprising an aqueous sodium hydroxide solution,
B2') continuously performing a neutralization reaction in a continuous flow reactor at a reaction temperature of 0°C,
B3') purifying compound of formula **(IV)** under continuous flow conditions,
C) combining the continuous flow of a solution containing the compound of formula **(IV)** with a continuous flow of a mixture comprising an oxidizing agent;
D) continuously producing a compound of formula **(V)** from the combined biphasic continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor.

The purification in step B3') can be performed by liquid-liquid extraction or phase separation.

The neutralization step after the esterification reaction and prior the oxidation step led to increased reaction yields. The removal of side products minimizes unwanted side reactions and facilitates purification of the final product. Excess sulfur dioxide from acid chloride formation is partitioned in the aqueous phase. Likewise, excess diol compound **(III)** can be removed, thereby minimizing unwanted aldehyde formation, which could hamper the reductive amination reaction at a later stage. Moreover, the removal of acidic side products facilitates obtaining ideal basic reaction conditions for the oxidation reaction of step D).

The oxidizing agent is selected from the group of oxidizing agents which oxidize primary alcohols to aldehydes. In a preferred embodiment of the present invention, the oxidation reaction of step D) is performed as a biphasic liquid-liquid reaction. Thus, the oxidizing agent consists of two immiscible liquids: i) an aqueous solution of oxidizing agent sodium hypochlorite, a base and an alkali bromide salt, and ii) an organic solution of a piperidin-1-oxyl nitroxyl radical compound and/or a pyrrolidine-1-oxyl nitroxyl radical compound as oxidation catalyst, preferably (2,2,6,6-tetramethylpiperidin-1-yl)oxyl in 2-methyl tetrahydrofuran. The aqueous solution of the oxidizing agent may be combined first with the organic solution of the oxidation catalyst and subsequently combined with the continuous flow obtained in step B'). Alternatively, the aqueous solution of the oxidizing agent may be combined simultaneously with the organic solution of the oxidation catalyst and with the continuous flow obtained in step B') by using a cross-shaped mixer or other suitable mixers.

Thus, the present invention is directed to a continuous flow process for producing a compound of general formula (I) comprising the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a biphasic mixture of an oxidizing agent;
D) continuously producing a compound of formula **(V)** from the combined biphasic continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor.

In other words, the present invention is directed to a continuous flow process for producing a compound of general formula **(I)** comprising the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with i) a continuous flow of a solution comprising an aqueous solution comprising sodium hypochlorite, a base and an alkali bromide salt, and ii) a continuous flow of a solution comprising an organic solution of a piperidin-1-oxyl nitroxyl radical compound and/or a pyrrolidine-1-oxyl nitroxyl radical compound;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor.

The present invention is also directed to a continuous flow process for producing a compound of general formula **(I)** comprising the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising sodium hypochlorite, a base, an alkali bromide salt, and a piperidin-1-oxyl nitroxyl radical compound and/or a pyrrolidine-1-oxyl nitroxyl radical compound;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor.

Preferably, the nitroxyl radical compound is (2,2,6,6-tetramethyl)piperidin-1-yl)oxyl. In a preferred embodiment the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
wherein the continuous flow of a mixture comprising an oxidizing agent of step C) is obtained by combining i) an aqueous solution comprising the oxidizing agent sodium hypochlorite, sodium bicarbonate and potassium bromide, and ii) a solution of (2,2,6,6-tetramethylpiperidin-1-yl)oxyl in 2-methyl tetrahydrofuran.

In a further embodiment, the continuous flow comprising the compound of formula **(V)** obtained in step D) is subjected to an intermediate purification before step E). Thus, the inventive continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
D') purifying compound of formula **(V)** under continuous flow conditions;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula (I) from the combined continuous flow obtained in step E) in a continuous flow reactor.

Preferably, the intermediate purification in step D') is performed by liquid-liquid extraction, such as an in-line membrane-based separation.

Surprisingly, the inventors have found that it is particularly advantageous to perform a biphasic liquid-liquid oxidation reaction in step D) followed by an intermediate purification to remove catalysts, side products and salts from the product compound **(V)** without dilution while maintaining a high overall productivity. Due to the compartmentation of catalysts, side products, salts and the product compound **(V)** in different phases, the intermediate purification is facilitated by the liquid-liquid reaction.

Thus, in a preferred embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a biphasic mixture of an oxidizing agent;
D) continuously producing a compound of formula **(V)** from the combined biphasic continuous flow obtained in step C) in a continuous flow reactor;
D') purifying compound of formula **(V)** under continuous flow conditions;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor.

Also, in a preferred embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with i) a continuous flow of a solution comprising an aqueous solution comprising sodium hypochlorite, a base and an alkali bromide salt, and ii) a continuous flow of a solution comprising an organic solution of a piperidin-1-oxyl nitroxyl radical compound and/or a pyrrolidine-1-oxyl nitroxyl radical compound;
D) continuously producing a compound of formula **(V)** from the combined biphasic continuous flow obtained in step C) in a continuous flow reactor;
D') purifying the compound of formula **(V)** under continuous flow conditions by liquid-liquid extraction;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor.

Also, in a preferred embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
B) continuously producing a compound of formula **(IV)** from the continuous flow of the solution comprising acid chloride **(II)** and diol compound **(III)** in a continuous flow reactor in the absence of a base;
B1') combining the continuous flow of a solution containing the compound of formula **(IV)** with a continuous flow comprising an aqueous base,
B2') continuously performing a neutralization reaction in a continuous flow reactor at a reaction temperature below 23°C,
B3') purifying compound of formula **(IV)** under continuous flow conditions,
C) combining the continuous flow of a solution containing the compound of formula **(IV)** with i) a continuous flow of a solution comprising an aqueous solution comprising sodium hypochlorite, a base and an alkali bromide salt, and ii) a continuous flow of a solution comprising an organic solution of a piperidin-1-oxyl nitroxyl radical compound and/or a pyrrolidine-1-oxyl nitroxyl radical compound;
D) continuously producing a compound of formula **(V)** from the combined biphasic continuous flow obtained in step C) in a continuous flow reactor;
D') purifying the compound of formula **(V)** under continuous flow conditions by liquid-liquid extraction;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor.

The inventors have further found that it is particularly beneficial to remove the hydrogen chloride formed in the esterification reaction of step B) by using a basic (or alkaline) biphasic continuous flow of an oxidizing agent in step C). Salt precipitation, which may occur due to the neutralization of the hydrogen chloride with a base is avoided since the salts dissolve in the aqueous phase, thereby maintaining a homogeneous reaction mixture, which does not hamper the continuous flow process and, more importantly, does not lower the reaction yield and selectivity of the esterification reaction. Additionally, the formed salts may be conveniently removed by an intermediate purification together with catalysts, side products and other salts from the product compound **(V)** without dilution while maintaining a high overall productivity.

Thus, in a preferred embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a basic biphasic continuous flow of an oxidizing agent;
C') neutralizing the continuous flow obtained in step C);
D) continuously producing a compound of formula **(V)** from the combined biphasic continuous flow obtained in step C)' in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor.

In a further preferred embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a basic biphasic continuous flow of an oxidizing agent;
C') neutralizing the continuous flow obtained in step C);
D) continuously producing a compound of formula **(V)** from the combined biphasic continuous flow obtained in step C) in a continuous flow reactor;
D') purifying the compound of formula **(V)** under continuous flow conditions;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor.

In a further preferred embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising sodium hypochlorite, a base, an alkali bromide salt, and a piperidin-1-oxyl nitroxyl radical compound and/or a pyrrolidine-1-oxyl nitroxyl radical compound;
C') neutralizing the continuous flow obtained in step C);
D) continuously producing a compound of formula **(V)** from the combined biphasic continuous flow obtained in step C') in a continuous flow reactor;
D') purifying the compound of formula **(V)** under continuous flow conditions by liquid-liquid extraction;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor.

Particularly preferred is an embodiment of the inventive continuous flow process, wherein the reducing agent for the reductive amination of the compound of formula **(V)** with amine compound of formula **(VI** in step F) is a soluble boron compound selected from: NaBH₄, NaBH₄/Celite/LiCl, NaCNBH₃, NaBH(OAc)₃, Me₄NBH(OAc)₃, or borane dimethylsulfide. Soluble boron compounds are preferred reducing agents since no heterogeneous reaction mixtures are formed, which could have a negative impact on the continuous flow process. Thus, in a further embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined biphasic continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor; wherein the reducing agent is a boron compound selected from: NaBH₄, NaBH₄/Celite/LiCl, NaCNBH₃, NaBH(OAc)₃, Me₄NBH(OAc)₃, or borane dimethylsulfide.

Preferably, the combined continuous flow obtained from step E) comprises at least one C₁-C₅ alcohol and an aprotic solvent.

Thus, in a further embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined biphasic continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
wherein the combined continuous flow obtained from step E) comprises at least one C₁-C₅ alcohol and an aprotic solvent.

Preferably, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined biphasic continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
wherein the combined continuous flow obtained from step E) comprises methanol and 2-methyl tetrahydrofuran.

In a preferred embodiment of the inventive continuous flow process the continuous flow containing the compound of formula **(V)** in step E) is combined simultaneously with the continuous flow of a solution comprising a reducing agent and with the continuous flow of a solution comprising an amine compound of formula **(VI)** using a cross-shaped mixer. Simultaneous mixing of the three continuous flows reduces the solid formation. Thus, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
E) simultaneously combining the continuous flow containing the compound of formula **(V)** obtained in step D) with a continuous flow of a solution comprising a reducing agent and with a continuous flow of a solution comprising an amine compound of formula **(VI)** by using a cross shaped mixer; and
F) continuously producing the compound of formula **(I)** from the combined continuous flow obtained in step E) in a continuous flow reactor; wherein the combined continuous flow obtained from step E) comprises methanol and 2-methyl tetrahydrofuran.

In a further embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor; and
G) isolating the compound of formula **(I)** produced in step F).

In a further embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
wherein R₃ represents -H and/or wherein X represents -OH.

In a further embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
wherein

| | |
|---|---|
| R₁ | represents -(CH₂)₅-CH₃; |
| R₂ | represents -(CH₂)₇-CH₃; |
| R₃ | represents -H; and |
| L | represents -(CH₂)₄-; |
| P | represents -(CH₂)₄-; and |
| X | represents -OH. |

A further aspect of the invention is directed to a continuous flow process for producing a vaccine composition comprising the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
G) isolating the compound of formula **(I)** produced in step F); and
G*) preparing a vaccine formulation containing the compound of formula **(I).**

In a preferred embodiment, the continuous flow process comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor; and
G*) preparing a vaccine formulation containing the compound of formula **(I).**

The term "continuous" as used herein means, for instance, that there is provided a flow of a solution or mixture containing the reaction components such as a compound of formula **(I)-(VII)** to the reactor which, is continuously converted while flowing through the reactor system in the direction from an inlet to an outlet such that a reaction product can be continuously derived at the outlet of the reactor without dividing the reaction mixture into parts. The term "continuous" or "continuously" in regard to the synthesis of alcohol compound of formula **(IV)** can be defined as a movement of the solution or mixture containing the compounds of formula **(II)** and **(III)** through the continuous flow reactor while being heated. This movement should only be in one direction, namely from the inlet to the outlet of a continuous flow reactor or in other words from a mixing device or from a reservoir containing the starting materials (e.g. containing the solution of acid chloride **(II)** and diol **(III)** or acid **(VII)** and a chlorinating agent) to the reactor. The movement could also temporarily stop (velocity of the movement is zero) during a part of the reaction time (residence time) in the reactor. Nevertheless, during the time of the reaction, there must be preferably a movement for a certain time in the direction described above.

The term "continuous" or "continuously" in regard to the synthesis of aldehyde compound of formula **(V)** can be defined as a movement of the solution or mixture containing the compound of formula **(IV)** and an oxidizing agent through the continuous flow reactor. This movement should only be in one direction, namely from the inlet to the outlet of a continuous flow reactor. The movement could also temporarily stop (velocity of the movement is zero) during a part of the reaction time (residence time) in the reactor. Nevertheless, during the time of the reaction, there must be preferably a movement for a certain time in the direction described above.

The term "continuous" or "continuously" in regard to the synthesis of cationic lipid of formula **(I)** can be defined as a movement of the solution or mixture containing the compound of formula **(V),** the amine compound of formula **(VI)** and a reducing agent through the continuous flow reactor. This movement should only be in one direction, namely from the inlet to the outlet of a continuous flow reactor. The movement could also temporarily stop (velocity of the movement is zero) during a part of the reaction time (residence time) in the reactor. Nevertheless, during the time of the reaction, there must be preferably a movement for a certain time in the direction described above.

It is preferred that acid chloride **(II)** is converted in a continuous manner to the cationic lipid of formula **(I).** Thus, in regard to the cationic lipid of formula **(I)** the term "continuous" or "continuously" refers to a movement of the solution or mixture containing acid chloride **(II)** and diol compound **(III)** through a first continuous flow reactor, wherein the esterification to alcohol **(IV)** takes place to a mixer for combining with an oxidizing agent, to a second continuous flow reactor, where the oxidation to aldehyde **(V)** takes place, to a mixer for combining with an amine **(VI)** and a reducing agent to a third continuous flow reactor for reductive amination of aldehyde **(V)** to compound of general formula **(I).** This movement should only be in one direction, namely from the inlet to the outlet of the first continuous flow reactor and then from the inlet to the outlet of the second continuous flow reactor to the inlet of the third continuous flow reactor. The movement could also temporarily stop (velocity of the movement is zero) during a part of the time of the esterification and/or oxidation and/or reductive amination reaction. Thus, during the time of the esterification and/or oxidation and/or reductive amination reaction, there must be a movement for a certain time in the direction described above. However although the flow of the reaction mixture might temporarily stop, the reaction mixture is processed through the continuous flow reactors in a manner that not parts of the reaction mixture are in between processed batch-wise. Thus there is a continuous flow through the continuous flow reactors, which might be processed with different velocities and might also stop but is not divided into single batches which are processed separately and later on probably combined again. Thus, if the continuous flow is theoretically divided into subsequent volume units it is still a continuous flow as long as the order of these theoretical volume units is not altered.

Thus, the continuous flow as described herein may occur at a steady or a fluctuating flow rate. In case of a fluctuating flow the reaction mixture may also stop intermediately or periodically, hence the flow rate may fall down to zero. However, if once stopped the continuous flow has to continue in the direction from the inlet to the outlet of the respective reactor. "Continuous" as used herein also means that a desired product such as compound of formula **(I)** can be provided steadily without the necessity of starting a novel experiment or batch in order to increase the amount of the desired product after the reaction took place. The reaction set-up and the reactor design allow a steadily increasing amount of product when starting material is provided without upscaling the reactor dimensions. "Continuous" further means that if a starting material is constantly provided and converted, the conversion compound is consistently produced.

Thus if the steps A), B), C), D), E) and F) and optionally G) are performed in a continuous manner, the term "continuous" refers to an endless flow of a solution or mixture containing compound **(I)** through the third continuous flow reactor.

Of course the flow through the continuous flow reactors is theoretically endless and in practice will end after a certain time when for example vessels have to be emptied or refilled, or parts of the continuous flow reactors have to be repaired or replaced or the reservoir of starting material is empty.

The continuous flow process according to the invention can be realized with any conventional continuous flow system known in the art. Any continuous flow reactor is suitable for the inventive process that allows reaction temperatures up to 250 °C. Thus, the inventive continuous flow process can be realized for instance with continuous flow reactors comprising of a PTFE tube and a heating system which may be an oven used in conventional gas chromatography systems and which allows temperatures between ambient temperature and 250°C. Alternatively, the reactor could be a stainless steel reactor for temperatures between ambient temperature and 350°C. The reactor can have any common form in the art, such as a tube, coil, cylinder, double-wall cylinder, multi-walled cylinder, tubing, duct, pipe, spiral, helix, spiral coil, zig-zag coil, board, fluidized bed, multi-layered fluidized bed, pool, vessel, tank, basin or the like.

The continuous flow process according to the invention may be realized with a system comprising three different continuous flow reactors: a first continuous flow reactor for esterification reaction of acid chloride **(II)** and diol **(III),** a second continuous flow reactor for oxidation to aldehyde **(V)** and a third continuous flow reactor for reductive amination to compound of formula **(I).** The continuous flow process according to the invention may also be realized with a radial synthesizer as disclosed in WO 2017/148874 A1, which allows multiple use of the same reactor in a multistep synthesis.

It is to be understood that in embodiments of the inventive process, which comprise the intermediate purification step D'), the system is equipped with a further module for intermediate purification. Any conventional purification technique for continuous flow conditions is suitable for the intermediate purification step D'), such as chromatography, crystallization, or liquid-liquid extraction. Particularly preferred is liquid-liquid extraction, in which an organic and an aqueous phase are separated from each other by flowing the input stream across a hydrophobic membrane within an inline liquid-liquid separator (which contains a hydrophobic PTFE membrane sandwiched between two halves of a separator chip). The separation of the phases is promoted by the cross-membrane pressure, which forces the organic phase through the hydrophobic membrane. The organic phase can be then fed into the system for the next reaction step.

Combining steps (or mixing steps) C) and E) can be realized with conventional mixers. The mixers are preferably but not exclusively static inline mixers and they consist of PTFE, glass, stainless steel or PVC. Homogeneity of the flow of reagents is ensured by alternating movement of the flow of reagents through the static mixers. The static mixers do not consist of moving parts and is therefore nearly maintenance free. The mixers may be placed upstream to the inlet of the continuous flow reactor in order to mix reagents prior passing them to the reactor.

The term **"C₁-₁₂ alkyl"** as used herein refers to a saturated linear or branched carbon chain consisting of 1 to 12 carbon atoms. Examples are -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -CH(CH₃)-C(CH₃)₃, -C₇H₁₅, -C₃H₆-C(CH₃)₃, -C₄H₈-CH(CH₃)₂, -C₈H₁₇, -C₄H₈-C(CH₃)₃, -C₅H₁₀-CH(CH₃)₂, -C₉H₁₉, -C₅H₁₀-C(CH₃)₃, -C₆H₁₂-CH(CH₃)₂, -C₁₀H₂₁, -CeH₁₂-C(CH₃)₃, -C₇H₁₄-CH(CH₃)₂, -C₁₁H₂₃, -C₇H₁₄-C(CH₃)₃, -C₈H₁₆-CH(CH₃)₂, -Cₗ₂H₂₅, -C₈H₁₆-C(CH₃)₃, and -C₉H₁₈-CH(CH₃)₂.

The term **"C₁-₂₄ alkyl"** as used herein refers to a saturated linear or branched carbon chain consisting of 1 to 24 carbon atoms. Examples are -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -CH(CH₃)-C(CH₃)₃, -C₇H₁₅, -C₃H₆-C(CH₃)₃, -C₄H₈-CH(CH₃)₂, -C₈H₁₇, -C₄H₈-C(CH₃)₃, -C₅H₁₀-CH(CH₃)₂, -C₉H₁₉, -C₅H₁₀-C(CH₃)₃, -C₆H₁₂-CH(CH₃)₂, -C₁₀H₂₁, -C₆H₁₂-C(CH₃)₃, -C₇H₁₄-CH(CH₃)₂, -C₁₁H₂₃, -C₇H₁₄-C(CH₃)₃, -C₈H₁₆-CH(CH₃)₂, -C₁₂H₂₅, -C₈H₁₆-C(CH₃)₃, -C₉H₁₈-CH(CH₃)₂, -C₁₃H₂₇, -C₉H₁₈-C(CH₃)₃, -C₁₀H₂₀-CH(CH₃)₂, -C₁₄H₂₉, -C₁₀H₂₀-C(CH₃)₃, -C₁₁H₂₂-CH(CH₃)₂, -C₁₅H₃₁, -C₁₁H₂₂-C(CH₃)₃, -C₁₂H₂₄-CH(CH₃)₂, -C₁₆H₃₃, -C₁₂H₂₄-C(CH₃)₃, -C₁₃H₂₆-CH(CH₃)₂, -C₁₇H₃₅, -C₁₃H₂₆-C(CH₃)₃, -C₁₄H₂₈-CH(CH₃)₂, -C₁₈H₃₇, -C₁₄H₂₈-C(CH₃)₃, -C₁₅H₃₀-CH(CH₃)₂, -C₁₉H₃₉, -C₁₅H₃₀-C(CH₃)₃, -C₁₆H₃₂-CH(CH₃)₂, -C₂₀H₄₁, -C₁₆H₃₂-C(CH₃)₃, -C₁₇H₃₄-CH(CH₃)₂, -C₂₁H₄₃, -C₁₇H₃₄-C(CH₃)₃, -C₁₈H₃₆-CH(CH₃)₂, -C₂₂H₄₅, -C₁₈H₃₆-C(CH₃)₃, -C₁₆H₃₈-CH(CH₃)₂, -C₂₃H₄₇, -C₁₉H₃₈-C(CH₃)₃, -C₂₀H₄₀-CH(CH₃)₂, -C₂₄H₄₉, -C₂₆H₄₆-C(CH₃)₃, and -C₂₁H₄₂-CH(CH₃)₂.

The term **"C₂-₂₄ alkenyl"** as used herein refers to a linear or branched carbon chain containing at least one double bond and consisting of 1 to 24 carbon atoms. Examples are: -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₂H₄-CH=CH-CH=CH₂, -CH₂-CH=CH-CH₂-CH=CH₂, -CH=CH-C₂H₄-CH=CH₂, -CH₂-CH=CH-CH=CH-CH₃, -CH=CH-CH₂-CH=CH-CH₃, -CH=CH-CH=CH-C₂H₅, -CH=CH-CH=C(CH₃)₂, and -CH=CH-CH=CH-CH=CH₂.

The term **"C₁-₁₂ alkylene"** as used herein refers to a saturated linear or branched hydrocarbon diradical chain consisting of 1 to 12 carbon atoms. Examples are -CH₂-, -C₂H₄-, -C₃H₆-, -C(CH₃)₂-, -C₄H₈-, -CH₂-C(CH₃)₂-, -CH(CH₃)-C₂H₄-, -C₅H₁₀-, -CH(CH₃)-C₃H₆- -CH₂-CH(CH₃)-C₂H₄-, -CH(CH₃)-C(CH₃)₂-, -C(CH₃)₂-C₂H₄-, -C(C₂H₅)₂- -C₂H₄-C(CH₃)₂-, -C₆H₁₂-, -C₃H₆-C(CH₃)₂-, -C₂H₄-CH(CH₃)-C₂H₄-, -CH(CH₃)-C₄H₆-, -CH₂-CH(CH₃)-C₃H₆-, -CH(CH₃)-CH₂-C(CH₃)₂-, -CH(CH₃)-CH(CH₃)-C₂H₄-, -CH₂-CH(CH₃)-C(CH₃)₂-, -CH₂-C(CH₃)₂-C₂H₄-, -C(CH₃)₂-C₃H₆-, -C(CH₃)₂-C(CH₃)₂-, -C₇H,₄-, -C₄H₆-C(CH₃)₂-, -C₈H,₆-, -C₅H₁₀-C(CH₃)₂- -C₉H₁₈-, -C₆H₁₂-C(CH₃)₂- -C₁₀H₂₀-, -C₇H₁₄-C(CH₃)₂-, -C₁₁H₂₂-, -C₈H₁₆-C(CH₃)₂-, -C₁₂H₂₄-, and -C₉H₁₈-C(CH₃)₂-.

The term **"C₂-₁₂ alkynylene"** as used herein refers to a linear or branched hydrocarbon diradical chain containing at least one triple bond. Examples are: -C≡C-, -C≡C-CH₂-, -CH₂-C≡C-, -C₂H₄-C≡C-, -CH₂-C≡C-CH₂-, -C≡C-C₂H₄-, -C₃H₆-C≡C-, -C₂H₄-C≡C-CH₂-, -CH₂-C≡C-C₂H₄-, -C≡C-C₃H₆-, -C₄H₈-C≡C-, -C₃H₆-C≡C-CH₂-, -C₂H₄-C≡C-C₂H₄-, -CH₂-C≡C-C₃H₆-, -C≡C-C₄H₈-, -C≡C-C≡C-, -CH₂-C≡C-C≡C-, -C≡C-C≡C-CH₂-, and -C(C≡CH)₂-.

The term **"C₂-₂₄ alkynylene"** as used herein refers to a linear or branched hydrocarbon diradical chain containing at least one triple bond and consisting of 2 to 24 carbon atoms.

The term **"C₃-₁₅ cycloalkylene"** as used herein refers to a stable non aromatic monocyclic or polycyclic hydrocarbon radical consisting solely of carbon and hydrogen atoms, which may include fused or bridged ring systems, having from three to fifteen carbon atoms, and which is saturated or unsaturated and attached to the rest of the molecule by a single bond. Monocyclic radicals include, for example, cyclopropyl, cyclobutyl, cyclopentyl, cyclohexyl, cycloheptyl, and cyclooctyl. Polycyclic radicals include, for example, adamantyl, norbornyl, decalinyl, 7,7 dimethyl bicyclo[2.2.1]heptanyl, and the like, wherein these cyclic residues can be substituted with 1 to 5 substituents selected from -F, -CI,-Br, -I, -CN, -NO₂, -OH, -CO₂H, -CO₂CH₃, -CO₂C₂H₅, -COCH₃, -SO₂CH₃, -SO₂CF₃,-NH₂, -NHCOCH₃, -NHSO₂CH₃, -NHSO₂CF₃, -NHCH₃, -N(CH₃)₂, -CH₂OH, -OCH₃,-OCHF₂, -OCF₃ and -CF₃. However it is clear to a skilled person that the term "can be substituted" refers to the replacement of a hydrogen atom by one of the said substituents. The carbon atom number of C₃-₁₅ refers only to the carbon atoms of the **cyclic residues** and does not include the carbon atoms of the said substituents.

The term **"C₃₋₈ cycloalkenylene**," as used herein, refers to a divalent carbocyclic nonaromatic group having from three to 8 carbon atoms. Non-limiting examples of the cycloalkenylene include cycloprop-1-en-1,2-diyl; cycloprop-2-en-1,1-diyl; cycloprop-2-en-1,2-diyl; cyclobut-1-en-1,2-diyl; cyclobut-1-en-1,3-diyl; cyclobut-1-en-1,4-diyl; cyclobut-2-en-1,1-diyl; cyclobut-2-en-1,4-diyl; cyclopent-1-en-1,2-diyl; cyclopent-1-en-1,3-diyl; cyclopent-1-en-1,4-diyl; cyclopent-1-en-1,5-diyl; cyclopent-2-en-1,1-diyl; cyclopent-2-en-1,4-diyl; cyclopent-2-en-1,5-diyl; cyclopent-3-en-1,1-diyl; cyclopent-1,3-dien-1,2-diyl; cyclopent-1,3-dien-1,3-diyl; cyclopent-1,3-dien-1,4-diyl; cyclopent-1,3-dien-1,5-diyl; cyclopent-1,3-dien-5,5-diyl; norbornadien-1,2-diyl; norbornadien-1,3-diyl; norbornadien-1,4-diyl; norbornadien-1,7-diyl; norbornadien-2,3-diyl; norbornadien-2,5-diyl; norbornadien-2,6-diyl; norbornadien-2,7-diyl; and norbornadien-7,7-diyl. The cycloalkenylene may be unsubstituted or substituted (e.g., optionally substituted cycloalkenylene) as described for C₃-₁₅ cycloalkylene.

The **reducing agent** is a compound or a mixture of compounds capable of reducing aldehydes in the presence of a primary amine to amines. The reducing agent is for instance a borane compound, a borohydride, hydrogen in combination with a hydrogenation catalyst (e.g. Pt/C, Pd/C) or an electrode in combination with a protic solvent (electrochemical reduction). Preferably, the reducing agent is selected from the group consisting of sodium borohydride (NaBH₄), lithium borohydride (LiBH₄), potassium borohydride (KBH₄), calcium borohydride (Ca(BH₄)₂), NaCNBH₃, NaBH(OAc)₃, Me₄NBH(OAc)₃ (tetramethylammonium triacetoxy borohydride), borane dimethylsulfide, hydrogen and a mixture of the afore-mentioned reducing agent. Preferred is tetramethylammonium triacetoxy borohydride.

The reducing agent also encompasses electrochemical reductions.

The reducing agent is preferably soluble in the solvent used for the reaction.

The **chlorinating agent** is a compound which converts acid of formula **(VII)** to an acid chloride of formula **(II).** Examples are thionyl chloride, oxalyl chloride, phosgene and phosgene derivatives, such as bis(trichloromethyl)carbonate.

The **oxidizing agent** is a compound or a mixture of compounds which converts primary alcohol of formula **(IV)** selectively to aldehyde of formula **(V).** Examples are pyridinium chlorochromate, sodium hypochlorite/sodium bicarbonate/potassium bromide/TEMPO ((2,2,6,6-tetramethylpiperidin-1-yl)oxyl) or dimethyl sulfoxide/oxalyl chloride/triethylamine.

Preferably, pyridinium chlorochromate is not used as oxidizing agent in the inventive continuous flow process.

The **nitroxyl radical compound** is a stable radical compound and a catalyst for the oxidation of primary alcohols to aldehydes. Examples are 2,2,5,5 alkyl substituted piperidin-1-oxyl and 2,2,6,6 alkyl substituted pyrrolidine-1-oxyl as well as derivatives thereof, such as TEMPO.

In a preferred embodiment, the continuous flow process is performed in an aprotic solvent. Examples for aprotic solvents are: CH₂Cl₂, CHCl₃, ClH₂CCH₂Cl, CHCl₂CHCl₂, CCl₂FCCl₂F, CH₃CN, Et₂O, tert-butylmethylether (MTBE), 1,2-dimethoxyethane, pentane, hexane, heptanes, petroleum ether, cyclopentane, cyclohexane, benzene, toluene, PEG 400, THF, 2-methyl tetrahydrofuran, 1,3-dioxane, 1,4-dioxane, dimethylformamide, dimethylacetamide, *N*-methyl pyrrolidinone (NMP) and mixtures thereof. A preferred aprotic solvent is 2-methyl tetrahydrofuran.

Thus, the present invention is directed to a continuous flow process for producing a compound of general formula **(I)** comprising the following steps:
A) providing a continuous flow of a solution comprising acid chloride **(II)** and diol compound **(III)** in an aprotic solvent;
B) continuously producing a compound of formula **(IV)** from the continuous flow of the solution comprising acid chloride **(II)** and diol compound **(III)** in a continuous flow reactor;
C) combining the continuous flow of a solution containing the compound of formula **(IV)** with a continuous flow of a mixture comprising an oxidizing agent in an aprotic solvent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI)** in an aprotic solvent; and
F) continuously producing the compound of formula **(I)** from the combined continuous flow obtained in step E) in a continuous flow reactor.

In a preferred embodiment, the continuous flow process for producing a compound of general formula **(I)** comprises the following steps:
A) providing a continuous flow of a solution comprising acid chloride **(II)** and diol compound **(III)** in an aprotic solvent;
B) continuously producing a compound of formula **(IV)** from the continuous flow of the solution comprising acid chloride **(II)** and diol compound **(III)** in a continuous flow reactor;
C) combining the continuous flow of a solution containing the compound of formula **(IV)** with a continuous flow of a mixture comprising an oxidizing agent in the aprotic solvent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI)** in the aprotic solvent; and
F) continuously producing the compound of formula **(I)** from the combined continuous flow obtained in step E) in a continuous flow reactor,
wherein the aprotic solvent is 2-methyl tetrahydrofuran.

In a preferred embodiment, the continuous flow process for producing a compound of general formula (I) comprises the following steps:
A) providing a continuous flow of a solution comprising acid chloride **(II)** and diol compound **(III)** in an aprotic solvent;
B) continuously producing a compound of formula **(IV)** from the continuous flow of the solution comprising acid chloride **(II)** and diol compound **(III)** in a continuous flow reactor;
C) combining the continuous flow of a solution containing the compound of formula **(IV)** with a continuous flow of a mixture comprising an oxidizing agent in the aprotic solvent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI)** in the aprotic solvent; and
F) continuously producing the compound of formula **(I)** from the combined continuous flow obtained in step E) in a continuous flow reactor,
wherein the aprotic solvent is 2-methyl tetrahydrofuran and the solution comprising a reducing agent in step E) is a solution of the reducing agent in *N*-methyl pyrrolidinone.

The inventive process can also be performed without a solvent exchange. Thus, steps A) to F) can be run in the same solvent or solvent mixture. In a preferred embodiment, the continuous flow process for producing a compound of general formula **(I)** comprises the following steps:
A) providing a continuous flow of a solution comprising acid chloride **(II)** and diol compound **(III)** in an aprotic solvent;
B) continuously producing a compound of formula **(IV)** from the continuous flow of the solution comprising acid chloride **(II)** and diol compound **(III)** in a continuous flow reactor;
C) combining the continuous flow of a solution containing the compound of formula **(IV)** with a continuous flow of a mixture comprising an oxidizing agent in the aprotic solvent;
D) continuously producing a compound of formula **(V)** from the combined continuous flow obtained in step C) in a continuous flow reactor;
E) combining the continuous flow containing the compound of formula (V) obtained in step D) with a continuous flow of a solution comprising a reducing agent and with a continuous flow of a solution comprising an amine compound of formula (VI) in the aprotic solvent; and
F) continuously producing the compound of formula (I) from the combined continuous flow obtained in step E) in a continuous flow reactor,
wherein in steps A) to F) the same aprotic solvent is used.

In one embodiment, the reductive amination step F) is performed in a solvent mixture of at least one C₁-C₅ alcohol and an aprotic solvent. The addition of at least one C₁-C₅ alcohol increases the solubility of the reducing agent and increases the reaction rate.

The expression "at least one" C₁-₅ alcohol indicates that also mixtures of C₁-₅ alcohol could be used.

The term "C₁-₅ alcohol" refers to any monool, diol, triol, tetraol or pentaol. Preferred examples of a C₁-C₅ alcohol are: CH₃OH, CH₃CH₂OH, CH₃CH₂CH₂OH, CH₃CH₂CH₂CH₂OH, CH₃CH₂CH₂CH₂CH₂OH, HOCH₂CH₂OH, HOCH₂CH₂CH₂OH, HOCH₂CH₂CH₂CH₂OH, HOCH₂CH₂CH₂CH₂CH₂OH, HOCH₂CH(OH)CH₂OH, HOCH₂CH(OH)CH₂CH₂OH, HOCH₂CH(OH)CH(OH)CH₃, HOCH₂CH(OH)CH(OH)CH₂OH, HOCH₂CH(OH)CH₂CH₂CH₂OH, HOCH₂CH₂CH(OH)CH₂CH₂OH, HOCH₂CH(OH)CH(OH)CH₂CH₂OH, HOCH₂CH(OH)CH₂CH(OH)CH₂OH, HOCH₂CH(OH)CH(OH)CH(OH)CH₂OH, HC(CH₂OH)₃, HO-C(CH₂OH)₃, and C(CH₂OH)₄.

It was found that a preferred molar ratio of C₁-₅ alcohol to reducing agent is in the range of 1.0:0.1 to 1.0:10.0, more preferred 1.0:0.3 to 1.0:5.0, even more preferred 1.0:0.4 to 1.0:3.0, and most preferred 1.0:0.5 to 1.0:1.0. It was also found that a preferred C₁-₅ alcohol is methanol, ethanol and a mixture thereof.

The molar ratio between acid chloride **(II)** and diol compound **(III)** in step A) is preferably between 1:1 and 1:10, more preferably between 1:1.1 and 1:9, more preferably between 1:1.2 and 1:8, more preferably between 1:1.3 and 1:7, more preferably between 1:1.5 and 1:5, more preferably between 1:2 and 1:4, more preferably between 1:2.5 and 1:3.5, and most preferably between 12.7 and 1:3.2. Most preferably, the molar ratio between acid chloride **(II)** and diol compound **(III)** in step A) is about 1:3.

The esterification reaction in step B) is preferably performed at a temperature between 40°C and 150°C, more preferably between 60°C and 120°C and most preferably between 70°C and 100°C. In one embodiment the esterification reaction is performed at 100 °C. In one embodiment the esterification reaction is performed at 80°C. In one embodiment the esterification reaction is performed at 150°C.

In a preferred embodiment the mixture comprising an oxidizing agent of step C) is obtained by combining i) an aqueous solution comprising the oxidizing agent sodium hypochlorite, sodium bicarbonate and potassium bromide, and ii) a solution of (2,2,6,6-tetramethylpiperidin-1-yl)oxyl in 2-methyl tetrahydrofuran. Preferably, the aqueous solution i) comprises sodium hypochlorite and sodium bicarbonate in a molar ratio between 6:1 and 7:1.

The oxidization reaction of step D) is preferably performed at a constant reaction temperature under cooling in a continuous flow reactor. More preferably, the oxidization reaction of step D) is performed at a reaction temperature of 23°C under cooling in a continuous flow reactor.

The oxidization reaction of step D) is preferably performed at a pH between 8.5 and 10.5, more preferably between 8.6 and 10.4, more preferably between 8.7 and 10.3, more preferably between 8.8 and 10.2, more preferably between 8.9 and 10.1, more preferably between 9.0 and 10.0, more preferably between 9.1 and 9.9, more preferably between 9.2 and 9.8, more preferably between 9.3 and 9.7 and most preferably between 9.4 and 9.6. In a preferred embodiment, the oxidization reaction of step D) is performed at a pH of 9.5.

The reductive amination reaction of step F) is preferably performed at a constant reaction temperature under cooling in a continuous flow reactor. More preferably, the reductive amination reaction of step F) is performed at a reaction temperature of 23°C under cooling in a continuous flow reactor.

In further preferred embodiments of the continuous flow process described herein R₃ represents -H; R₁ and R₂ independently of each other represent C₁₋₂₄ alkyl; and X represents -OH.

In further preferred embodiments of the continuous flow process described herein R₃ represents -H; R₁ and R₂ independently of each other represent C₁₋₂₄ alkyl; P represents C₁₋₁₂ alkylene; and X represents -OH.

In further preferred embodiments of the continuous flow process described herein R₃ represents -H; R₁ and R₂ independently of each other represent C₁₋₂₄ alkyl; L represents -(CH₂-O-CH₂)₁₋₆-; and X represents -OH.

In further preferred embodiments of the continuous flow process described herein R₃ represents -H; R₁ and R₂ independently of each other represent C₁₋₂₄ alkyl; P represents -(CH₂)₁-(CH₂-O-CH₂)₁₋₆-(CH₂)₁-; and X represents -OH.

In further preferred embodiments of the continuous flow process described herein R₃ represents -H; R₁ and R₂ independently of each other represent C₁₋₂₄ alkyl; L represents C₁₋₁₂ alkylene; P represents -(CH₂)₁-(CH₂-O-CH₂) ₁₋₆-(CH₂)₁- ; and X represents -OH.

In further preferred embodiments of the continuous flow process described herein R₃ represents -H; R₁ and R₂ independently of each other represent C₁₋₂₄ alkyl; L represents C₁₋₁₂ alkylene; P represents C₁₋₁₂ alkylene; and X represents -OH.

In further preferred embodiments of the continuous flow process described herein R₃ represents -H; R₁ and R₂ independently of each other represent C₁₋₂₄ alkyl; L represents C₁₋₁₂ alkylene; and X represents -N(CH₃)₂.

A further aspect of the present invention is directed to a continuous flow process for producing ((4-hydroxybutyl)azanediyl)bis(hexane-6,1-diyl)bis(2-hexyldecanoate), thus the compound of general formula (I) wherein

| | |
|---|---|
| R₁ | represents -(CH₂)₅-CH₃; |
| R₂ | represents -(CH₂)₇-CH₃; |
| R₃ | represents -H; and |
| L | represents -(CH₂)₄-; |
| P | represents -(CH₂)₄-; and |
| X | represents -OH. |

Thus, the present invention is also directed to a continuous flow process for producing a compound of general formula **(I)** comprising steps A1) and A2) instead of step A):
A1) providing a continuous flow of a solution comprising a carboxylic acid of formula **(VII)** and a chlorinating agent;
A2) continuously producing an acid chloride of formula **(IV)** from the continuous flow of the solution comprising the carboxylic acid of formula **(VII)** and the chlorinating agent in a continuous flow reactor;
B) continuously producing a compound of formula **(IV)** from the continuous flow of the solution comprising acid chloride **(II)** and diol compound **(III)** in a continuous flow reactor;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor,
wherein R₁ represents -(CH₂)₅-CH₃; R₂ represents -(CH₂)₇-CH₃; R₃ represents -H ; and L represents -(CH₂)₄-; P represents -(CH₂)₄-; and X represents -OH .

Preferably, the chlorinating agent is selected from thionyl chloride, oxalyl chloride, bis(trichloromethyl)carbonate or phosgene. Most preferably, the chlorinating agent is thionyl chloride. Preferably, step A2) is performed at a reaction temperature between 23°C and 100°C more preferably at a reaction temperature between 40°C and 100°C, even more preferably at a reaction temperature between 60°C and 90°C and most preferably at a reaction temperature of about 80°C. Most preferably, the chlorinating agent is thionyl chloride and step A2) is performed at a reaction temperature of about 80°C.

In one embodiment, preferably 0.005 molar equivalents to 0.2 molar equivalents of N,N-dimethylformamide, more preferably 0.007 molar equivalents to 0.15 molar equivalents of *N*,*N*-dimethylformamide, more preferably 0.01 molar equivalents to 0.1 molar equivalents of *N*,*N*-dimethylformamide, and most preferably 0.05 molar equivalents of N,N-dimethylformamide are added in step A2) in order to increase the reaction rate.

Another embodiment of the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
B1') combining the continuous flow of a solution containing the compound of formula **(IV)** with a continuous flow comprising an aqueous base,
B2') continuously performing a neutralization reaction in a continuous flow reactor at a reaction temperature below 23°C,
B3') purifying compound of formula **(IV)** under continuous flow conditions,
C) combining the continuous flow of a solution containing the compound of formula **(IV)** with a continuous flow of a mixture comprising an oxidizing agent;
D) continuously producing a compound of formula **(V)** from the combined biphasic continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor
wherein R₁ represents -(CH₂)₅-CH₃; R₂ represents -(CH₂)₇-CH₃; R₃ represents -H ; and L represents -(CH₂)₄-; P represents -(CH₂)₄-; and X represents -OH .

Preferably, the aqueous base is an aqueous solution of an alkali metal hydroxide. Preferably, the aqueous base is a 1 molar aqueous solution of an alkali metal hydroxide. Preferably, the aqueous base is an aqueous solution of lithium hydroxide, sodium hydroxide or potassium hydroxide. Most preferably, the aqueous base is a 1 molar aqueous solution of sodium hydroxide. Preferably, step B3') is performed at a reaction temperature of 0 °C.

Another embodiment of the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor;
C) combining the continuous flow of a solution containing the compound of formula **(IV)** with a continuous flow of a mixture comprising an oxidizing agent and a base;
C') neutralizing the continuous flow obtained in step C);
D) continuously producing a compound of formula **(V)** from the combined continuous flow obtained in step C') in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
wherein R₁ represents -(CH₂)₅-CH₃; R₂ represents -(CH₂)₇-CH₃; R₃ represents -H ; and L represents -(CH₂)₄-; P represents -(CH₂)₄-; and X represents -OH.

In other words, the present invention is directed to a continuous flow process for producing a compound of general formula **(I)** comprising the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor, thereby forming hydrogen chloride;
C) combining the continuous flow of a solution containing the compound of formula **(IV)** with a continuous flow of a mixture comprising an oxidizing agent and a base;
C') neutralizing the hydrogen chloride formed in step B);
D) continuously producing a compound of formula **(V)** from the combined continuous flow obtained in step C') in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH.

The present invention is directed to a continuous flow process for producing a compound of general formula **(I)** comprising the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a biphasic mixture of an oxidizing agent;
D) continuously producing a compound of formula **(V)** from the combined biphasic continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH.

In other words, the present invention is directed to a continuous flow process for producing a compound of general formula **(I)** comprising the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with i) a continuous flow of a solution comprising an aqueous solution comprising sodium hypochlorite, a base and an alkali bromide salt, and ii) a continuous flow of a solution comprising an organic solution of a piperidin-1-oxyl nitroxyl radical compound and/or a pyrrolidine-1-oxyl nitroxyl radical compound;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH.

The present invention is also directed to a continuous flow process for producing a compound of general formula **(I)** comprising the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising sodium hypochlorite, a base, an alkali bromide salt, and a piperidin-1-oxyl nitroxyl radical compound and/or a pyrrolidine-1-oxyl nitroxyl radical compound;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH.

Preferably, the nitroxyl radical compound is (2,2,6,6-tetramethyl)piperidin-1-yl)oxyl. In a preferred embodiment the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
wherein the continuous flow of a mixture comprising an oxidizing agent of step C) is obtained by combining i) an aqueous solution comprising the oxidizing agent sodium hypochlorite, sodium bicarbonate and potassium bromide, and ii) a solution of (2,2,6,6-tetramethylpiperidin-1-yl)oxyl in 2-methyl tetrahydrofuran; and wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH.

In a further embodiment, the continuous flow comprising the compound of formula **(V)** obtained in step D) is subjected to an intermediate purification before step E). Thus, the inventive continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
D') purifying compound of formula **(V)** under continuous flow conditions;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula (I) from the combined continuous flow obtained in step E) in a continuous flow reactor;
wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH. Preferably, the intermediate purification in step D') is performed by liquid-liquid extraction, such as an in-line membrane-based separation.

Thus, in a preferred embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a biphasic mixture of an oxidizing agent;
D) continuously producing a compound of formula **(V)** from the combined biphasic continuous flow obtained in step C) in a continuous flow reactor;
D') purifying compound of formula **(V)** under continuous flow conditions;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH.

Also, in a preferred embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with i) a continuous flow of a solution comprising an aqueous solution comprising sodium hypochlorite, a base and an alkali bromide salt, and ii) a continuous flow of a solution comprising an organic solution of a piperidin-1-oxyl nitroxyl radical compound and/or a pyrrolidine-1-oxyl nitroxyl radical compound;
D) continuously producing a compound of formula **(V)** from the combined biphasic continuous flow obtained in step C) in a continuous flow reactor;
D') purifying the compound of formula **(V)** under continuous flow conditions by liquid-liquid extraction;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH.

Also, in a preferred embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
B) continuously producing a compound of formula **(IV)** from the continuous flow of the solution comprising acid chloride **(II)** and diol compound **(III)** in a continuous flow reactor in the absence of a base;
B1') combining the continuous flow of a solution containing the compound of formula **(IV)** with a continuous flow comprising an aqueous base,
B2') continuously performing a neutralization reaction in a continuous flow reactor at a reaction temperature below 23°C,
B3') purifying compound of formula **(IV)** under continuous flow conditions,
C) combining the continuous flow of a solution containing the compound of formula **(IV)** with i) a continuous flow of a solution comprising an aqueous solution comprising sodium hypochlorite, a base and an alkali bromide salt, and ii) a continuous flow of a solution comprising an organic solution of a piperidin-1-oxyl nitroxyl radical compound and/or a pyrrolidine-1-oxyl nitroxyl radical compound;
D) continuously producing a compound of formula **(V)** from the combined biphasic continuous flow obtained in step C) in a continuous flow reactor;
D') purifying the compound of formula **(V)** under continuous flow conditions by liquid-liquid extraction;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor,
wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH.

In a preferred embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a basic biphasic continuous flow of an oxidizing agent;
C') neutralizing the continuous flow obtained in step C);
D) continuously producing a compound of formula **(V)** from the combined biphasic continuous flow obtained in step C)' in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH.

In a further preferred embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a basic biphasic continuous flow of an oxidizing agent;
C') neutralizing the continuous flow obtained in step C);
D) continuously producing a compound of formula **(V)** from the combined biphasic continuous flow obtained in step C) in a continuous flow reactor;
D') purifying the compound of formula **(V)** under continuous flow conditions;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH.

In a further preferred embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising sodium hypochlorite, a base, an alkali bromide salt, and a piperidin-1-oxyl nitroxyl radical compound and/or a pyrrolidine-1-oxyl nitroxyl radical compound;
C') neutralizing the continuous flow obtained in step C);
D) continuously producing a compound of formula **(V)** from the combined biphasic continuous flow obtained in step C') in a continuous flow reactor;
D') purifying the compound of formula **(V)** under continuous flow conditions by liquid-liquid extraction;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH.

In a further embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor at a temperature between 40 °C and 150 °C in the absence of a base;
C) combining the continuous flow of a solution containing the compound of formula **(IV)** with a continuous flow of a mixture comprising an oxidizing agent;
D) continuously producing a compound of formula **(V)** from the combined biphasic continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH. Preferably, the esterification reaction in step B) is performed at 100°C.

In a further embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined biphasic continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
wherein the reducing agent is a boron compound selected from: NaBH₄, NaBH₄/Celite/LiCl, NaCNBH₃, NaBH(OAc)₃, Me₄NBH(OAc)₃, or borane dimethylsulfide; and
wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH.

Preferably, the combined continuous flow obtained from step E) comprises at least one C₁-C₅ alcohol and an aprotic solvent. Thus, in a further embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined biphasic continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor; wherein the combined continuous flow obtained from step E) comprises at least one C₁-C₅ alcohol and an aprotic solvent;
wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH.

Preferably, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined biphasic continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor;
wherein the combined continuous flow obtained from step E) comprises methanol and 2-methyl tetrahydrofuran;
wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH.

In a preferred embodiment of the inventive continuous flow process the continuous flow containing the compound of formula **(V)** in step E) is combined simultaneously with the continuous flow of a solution comprising a reducing agent and with the continuous flow of a solution comprising an amine compound of formula **(VI)** using a cross-shaped mixer. Simultaneous mixing of the three continuous flows reduces the solid formation. Thus, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
E) simultaneously combining the continuous flow containing the compound of formula **(V)** obtained in step D) with a continuous flow of a solution comprising a reducing agent and with a continuous flow of a solution comprising an amine compound of formula **(VI)** by using a cross shaped mixer; and
F) continuously producing the compound of formula **(I)** from the combined continuous flow obtained in step E) in a continuous flow reactor;
wherein the combined continuous flow obtained from step E) comprises methanol and 2-methyl tetrahydrofuran;
wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH.

In a further embodiment, the continuous flow process for producing a compound of general formula **(I)** comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor; and
G) isolating the compound of formula **(I)** produced in step F);
wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH.

A further aspect of the invention is directed to a continuous flow process for producing a vaccine composition comprising the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula (I) from the combined continuous flow obtained in step E) in a continuous flow reactor;
G) isolating the compound of formula **(I)** produced in step F); and
G*) preparing a vaccine formulation containing the compound of formula **(I);**
wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH.

In a preferred embodiment, the continuous flow process comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor; and
G*) preparing a vaccine formulation containing the compound of formula **(I);**
wherein R₁ represents -(CH₂)₅-CH₃; R₂ represents -(CH₂)₇-CH₃; R₃ represents -H; and L represents -(CH₂)₄-; P represents -(CH₂)₄-; and X represents -OH.

### Vaccine formulation

Another aspect of the present invention is directed to a method for preparing a vaccine formulation containing the compound of formula **(I).** The compound of formula **(I)** as a cationic lipid is particularly useful for forming lipid nanoparticles together with other lipid components that facilitate delivery of therapeutic nucleic acids, particularly mRNA. The term "immunogenic mRNA" refers to mRNA that encodes the viral Spike (S) glycoprotein of SARS-CoV-2 virus.

Thus, the present invention is also directed to a continuous flow process comprising the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor; and
G*) preparing a vaccine formulation comprising an immunogenic mRNA encapsulated in a lipid nanoparticle comprising the compound of formula **(I).**

In a preferred embodiment, the continuous flow process comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
B1') combining the continuous flow of a solution containing the compound of formula **(IV)** with a continuous flow comprising an aqueous base,
B2') continuously performing a neutralization reaction in a continuous flow reactor at a reaction temperature below 23°C,
B3') purifying compound of formula **(IV)** under continuous flow conditions,
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor; and
G*) preparing a vaccine formulation comprising an immunogenic mRNA encapsulated in a lipid nanoparticle comprising the compound of formula **(I).**

In a further preferred embodiment, the continuous flow process comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
B1') combining the continuous flow of a solution containing the compound of formula **(IV)** with a continuous flow comprising an aqueous base,
B2') continuously performing a neutralization reaction in a continuous flow reactor at a reaction temperature below 23°C,
B3') purifying compound of formula **(IV)** under continuous flow conditions,
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
D') purifying the compound of formula **(V)** under continuous flow conditions;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor; and
G*) preparing a vaccine formulation comprising an immunogenic mRNA encapsulated in a lipid nanoparticle comprising the compound of formula **(I).**

Preferably, the intermediate purifications in step B3') and step D') are performed by liquid-liquid extraction, such as an in-line membrane-based separation.

In a further preferred embodiment, the continuous flow process comprises the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent and a base;
C') neutralizing the hydrogen chloride formed in step B);
D) continuously producing a compound of formula **(V)** from the combined continuous flow obtained in step C) in a continuous flow reactor;
D') optionally purifying the compound of formula **(V)** under continuous flow conditions;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor; and
G*) preparing a vaccine formulation comprising an immunogenic mRNA encapsulated in a lipid nanoparticle comprising the compound of formula **(I).**

In preferred embodiments the lipid nanoparticle comprises a cationic lipid of formula **(I);** a neutral lipid; a steroid; a polymer conjugated lipid; and an immunogenic mRNA encapsulated within or associated with the lipid nanoparticle.

Preferably, the lipid nanoparticle comprises the cationic lipid of formula **(I)** in the amount of 40 and 50 mol percent. Preferably, the neutral lipid is 1,2-Distearoyl-sn-glycero-3-phosphocholine (DSPC). Preferably, the steroid is cholesterol. Preferably, the polymer conjugated lipid is 2-[(polyethylene glycol)-2000]-N,N ditetradecylacetamide. In a preferred embodiment, the lipid nanoparticle comprises 40 and 50 mol percent of the cationic lipid of formula **(I),** 1,2-Distearoyl-sn-glycero-3-phosphocholine (DSPC), cholesterol, 2-[(polyethylene glycol)-2000]-N,N ditetradecylacetamide and an immunogenic mRNA.

The lipid nanoparticles may be formed by any suitable method known in the art. To this extent, the lipid components cationic lipid of formula (I); neutral lipid; steroid; and polymer conjugated lipid; are dissolved in at least one C₁-₅ alcohol, preferably ethanol. The solution containing the lipid components is then mixed with an aqueous solution of the immunogenic mRNA to obtain the lipid nanoparticle with an encapsulated or associated immunogenic mRNA.

Thus, the present invention is also directed to a continuous flow process comprising the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula **(I)** from the combined continuous flow obtained in step E) in a continuous flow reactor; and
G1*) preparing a solution of lipid components, wherein one lipid component is the compound of formula **(I);**
G2*) mixing the solution of lipid components with an aqueous solution of an immunogenic mRNA in order to obtain a vaccine formulation comprising the immunogenic mRNA encapsulated in a lipid nanoparticle comprising the compound of formula **(I).**

Preferably, the present invention is also directed to a continuous flow process comprising the following steps:
A) providing a continuous flow of a solution comprising acid chloride **(II)** and diol compound **(III);**
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula **(IV)** with a continuous flow of a mixture comprising an oxidizing agent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI);** and
F) continuously producing the compound of formula (I) from the combined continuous flow obtained in step E) in a continuous flow reactor; and
G1*) preparing a solution of the cationic lipid of formula (I); a neutral lipid; a steroid; and a polymer conjugated lipid in ethanol;
G2*) mixing the solution obtained in step G1*) with an aqueous solution of an immunogenic mRNA in order to obtain a vaccine formulation comprising the immunogenic mRNA encapsulated in a lipid nanoparticle comprising the compound of formula **(I)**.

The solution containing the lipid components can be mixed with the aqueous solution of the immunogenic mRNA according to any conventional mixing method, including T-junction mixing, microfluidic hydrodynamic focusing and staggered herringbone mixing. Preferably, the T-junction mixing is used in the processes described herein. A T-junction mixer provides a controlled mixing environment compared to macroscopic mixing methods (e.g., vortexing or pipetting), thereby leading to reproducible production of lipid particles. The mixing occurs when the two input streams (lipid components and aqueous mRNA solution) in the T-junction collide, resulting in a turbulent output flow containing the lipid nanoparticles.

Thus, the present invention is also directed to a continuous flow process comprising the following steps:
A) providing a continuous flow of a solution comprising acid chloride **(II)** and diol compound **(III)**;
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula (**IV**) with a continuous flow of a mixture comprising an oxidizing agent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI)**; and
F) continuously producing the compound of formula (**I**) from the combined continuous flow obtained in step E) in a continuous flow reactor; and
G1*) preparing a solution of the cationic lipid of formula (**I**); a neutral lipid; a steroid; and a polymer conjugated lipid in ethanol;
G2*) rapid mixing the solution obtained in step G1*) with an aqueous solution of an immunogenic mRNA by using an in-line T-junction mixer in order to obtain a vaccine formulation comprising the immunogenic mRNA encapsulated in a lipid nanoparticle comprising the compound of formula **(I)**.

To obtain a sterile vaccine formulation for in vivo application the vaccine formulation may be subjected to sterile filtration. Thus, the present invention is also directed to a continuous flow process comprising the following steps:
A) providing a continuous flow of a solution comprising acid chloride **(II)** and diol compound **(III)**;
B) continuously producing a compound of formula **(IV)** from the continuous flow of the solution comprising acid chloride **(II)** and diol compound **(III)** in a continuous flow reactor in the absence of a base;
C) combining the continuous flow of a solution containing the compound of formula (**IV**) with a continuous flow of a mixture comprising an oxidizing agent;
D) continuously producing a compound of formula **(V)** from the combined continuous flow obtained in step C) in a continuous flow reactor;
E) combining the continuous flow containing the compound of formula **(V)** obtained in step D) with a continuous flow of a solution comprising a reducing agent and with a continuous flow of a solution comprising an amine compound of formula **(VI)**; and
F) continuously producing the compound of formula (**I**) from the combined continuous flow obtained in step E) in a continuous flow reactor; and
G1*) preparing a solution of the cationic lipid of formula (**I**); a neutral lipid; a steroid; and a polymer conjugated lipid in ethanol;
G2*) mixing the solution obtained in step G1*) with an aqueous solution of an immunogenic mRNA in order to obtain a vaccine formulation comprising the immunogenic mRNA encapsulated in a lipid nanoparticle comprising the compound of formula **(I)**;
G3*) sterile filtration of the vaccine formulation obtained in step G2*).

Optionally, adjuvants, buffers, excipients and/or diluents may be added to the vaccine formulation in order to improve immunological activity, stability and bring it into an administrable form.

The term "adjuvant" as used herein refers to an immunological adjuvant i.e. a material used in a vaccine composition that modifies or augments the effects of said vaccine by enhancing the immune response to a given antigen contained in the vaccine without being antigenically related to it. For the persons skilled in the art, classically recognized examples immunological adjuvants include but are not restricted to oil emulsions (e.g. Freund's adjuvant), saponins, aluminium or calcium salts (e.g. alum), non-ionic block polymer surfactants, and many others.

The vaccine formulation may be administered subcutaneously, by spray, by injection, orally, intraocularly, intramuscular, intratracheally or nasally. Preferably, the vaccine formulation is administered intramuscular.

Preferably the vaccine formulation is formulated as a liquid buffer solution. Preferred buffers are potassium dihydrogen phosphate, disodium hydrogen phosphate and tris (tormethamine) to maintain the pH between 7 and 8.

Further suitable excipients are potassium chloride, sodium chloride, sodium acetate, sucrose and water for injection (diluent).

### Description of Figures

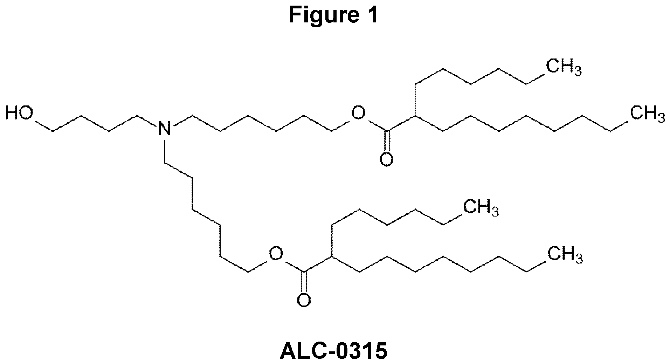

- **Fig. 1:**: shows the structure of cationic lipid ALC-0315.

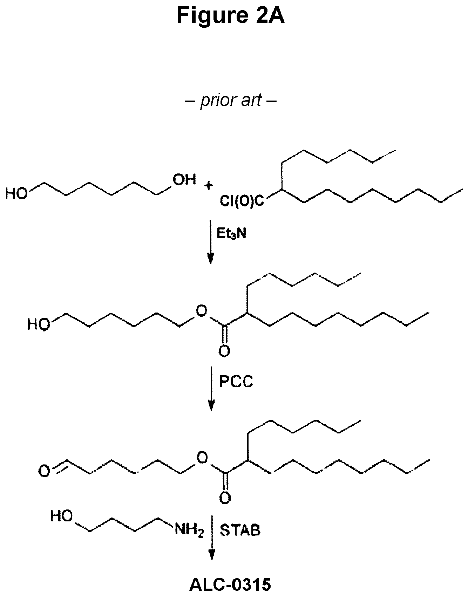

- **Fig. 2A:**: shows the synthesis of ALC-0315 known from WO 2016/176330 A1. In a first step, 2-hexyldecanoyl chloride is esterified with 1,6-hexanediol in the presence of 2 equiv. triethylamine. The remaining free alcohol group is then oxidized with toxic pyridinium chlorochromate to the corresponding aldehyde, which is in a last step reduced with sodium triacetoxy borohydride and acetic acid in the presence of 4-amino-1-butanol to ALC-0315.

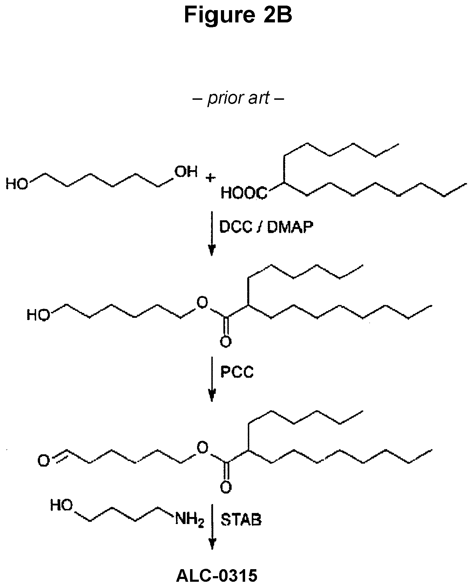

- **Fig. 2B:**: shows the synthesis of ALC-0315 known from WO 2017/075531 A1. In a first step, 2-hexyldecanoic acid is esterified with 1,6-hexanediol in the presence of dicyclohexylcarbodiimide and 4-dimethylaminopyridine. The remaining free alcohol group is then oxidized with toxic pyridinium chlorochromate to the corresponding aldehyde, which is in a last step reduced with sodium triacetoxy borohydride and acetic acid in the presence of 4-amino-1-butanol to ALC-0315.
- **Fig. 3:**: Schematic drawing of a synthesis of compound of formula (**I**) according to the invention in continuous manner.
- **Fig. 4:**: Schematic drawing of a synthesis of compound of formula (**I**) according to the invention in continuous manner.
- **Fig. 5:**: Schematic drawing of a synthesis of compound of formula (**I**) according to the invention in continuous manner using biphasic TEMPO/sodium hypochlorite/KBr as oxidizing agent and two phase separation steps.
- **Fig. 6:**: shows HPLC chromatogram of the crude reaction mixture of the esterification reaction in Example 3. The chromatogram shows a 6-hydroxyhexyl 2-hexyldecanoate/hexane-1,6-diyl bis(2-hexyldecanoate) ratio of 8:1

### Examples

### Example 1: Continuous preparation of ALC-0315

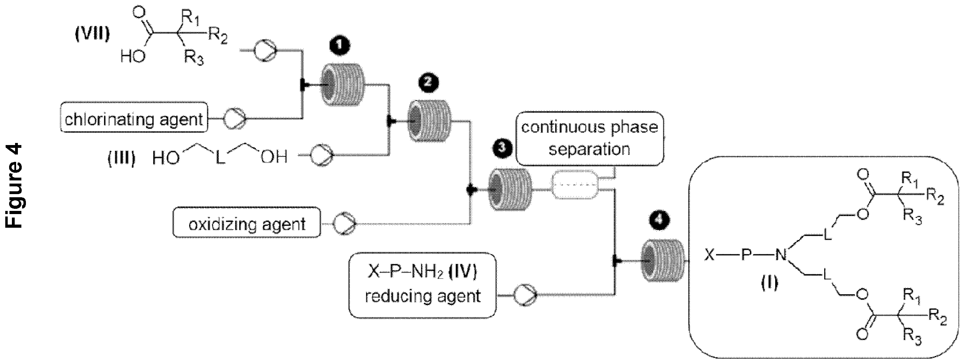

The synthesis of lipid ALC-0315 was performed in a four-step continuous flow process starting from 2-hexyldecanoic acid as shown in **Figure 4****.**

First, 2-hexyldecanoic acid is converted to 2-hexyldecanoyl chloride in 2-methyl tetrahydrofuran using thionyl chloride in a flow reactor for heating (1) at temperatures ranging from 23 to 80 °C. A faster conversion to the acid chloride was observed by addition of 0.01 molar equivalents of *N*,*N*-dimethylformamide. The esterification of 2-hexyldecanoyl chloride using excess amounts of 1,6-hexanediol was carried out at elevated temperature without addition of a base (2). The mono-ester, 6-hydroxyhexyl 2-hexyldecanoate is oxidized in the third step (3) via a biphasic reaction using an aqueous solution of sodium hypochlorite, a basic aqueous solution of potassium bromide, and a solution of 2,2,6,6-tetramethylpiperidinyloxyl (TEMPO) in 2-methyl tetrahydrofuran. The product was purified using an in-line liquid-liquid separator (3). In the final synthetic step, the aldehyde stream is combined with a methanolic solution of 4-amino-1-butanol and a borohydride, producing ((4-hydroxybutyl)azanediyl)bis(hexane-6,1-diyl) bis(2-hexyldecanoate) (4).

### Example 2: Unsuccessful attempts of continuously preparing ALC-0315

The synthesis of lipid ALC-0315 was attempted in a four-step continuous flow process starting from 2-hexyldecanoic acid similar to **Example 1.** The second step, the esterification reaction, was performed differently with 2 equivalents of triethylamine. The formation of a heterogeneous reaction mixture was observed. The final product ((4-hydroxybutyl)azanediyl)bis(hexane-6,1-diyl) bis(2-hexyldecanoate) was obtained in significantly lower yields compared to **Example 1.**

### Example 3: Continuous preparation of ALC-0315 using aqueous sodium hydroxide solution as base for esterification reaction

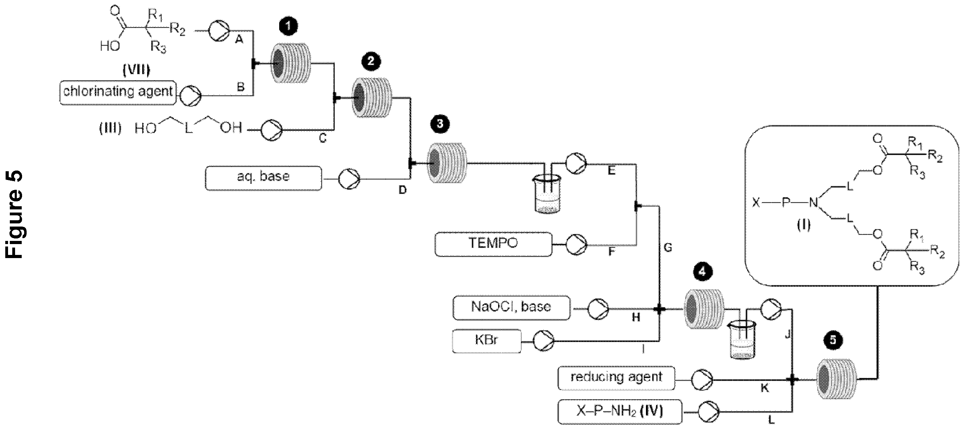

The synthesis of lipid ALC-0315 was performed in a four-step continuous flow process starting from 2-hexyldecanoic acid as shown in **Figure 5****.** A solution of 2-hexyldecanoic acid (35.23 g, 137.4 mmol) and *N*,*N*-dimethylformamide (502 mg, 6.87 mmol) in 2-methyltetrahydrofuran (1.374 M) was pumped through 1/16" Tefzel^{™} tubing (I.D. 1 mm) using an HPLC pump at a flow rate of 0.901 mL min⁻¹ [Line A]. Neat thionyl chloride (18.00 g, 151.3 mmol) was pumped through Tefzel^{™} tubing (I.D. 1 mm) using a continuous syringe pump at a flow rate of 0.099 mL min⁻¹ [Line B]. Line B was combined with Line A via a T-mixer providing a continuous flow of a solution which was heated at 80 °C in a 20 mL reactor (Reactor 1, 20 min residence time).

A solution of hexan-1,6-diol (48.71 g, 412.2 mmol) in 2-methyltetrahydrofuran (2.50 M) was pumped through 1/16" Tefzel^{™} tubing (I.D. 1 mm) using an HPLC pump at a flow rate of 1.5 mL min⁻¹ [Line C]. Line C was combined with the output from Reactor 1 via a T-mixer providing a continuous flow of a solution, which was heated at 100 °C in a 10 mL reactor (Reactor 2, 4 min residence time). A solution of sodium hydroxide in water (1 M) was pumped through 1/16" Tefzel^{™} tubing (I.D. 1 mm) using an HPLC pump at a flow rate of 5.5 mL min⁻¹ [Line D]. Line D was combined with the output from Reactor 2 via a T-mixer providing a continuous flow of a solution, which was cooled at 0 °C in a 10 mL reactor (Reactor 3, 1.25 min residence time).

The entire system was held under constant pressure using a backpressure regulator (e.g. 2.8 bar, IDEX 40 PSI, P-761).

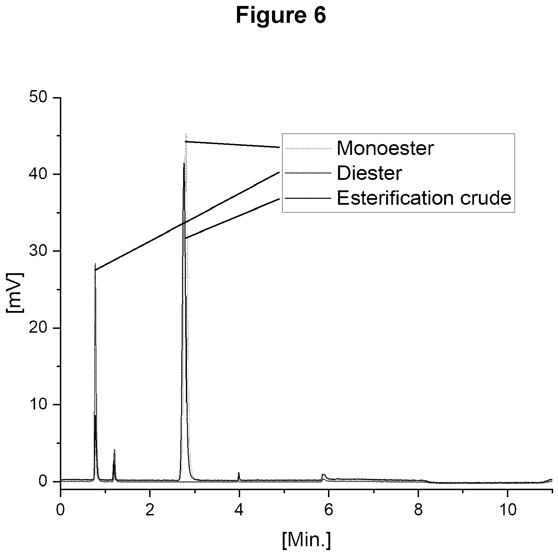

After a steady state was reached (2 residence times, 50.5 min), the biphasic outlet was collected for 30 min and phases separated. The organic phase was used in the next step without any further purification. For analytical purposes, an aliquot (5 mL) of the organic phase (47 mL) was analysed by HPLC and NMR and purified by column chromatography. HPLC analysis of the crude mixture showed a 6-hydroxyhexyl 2-hexyldecanoate/hexane-1,6-diyl bis(2-hexyldecanoate) ratio of 8:1 (**Figure 6**). As a representative example, from 1.603 g of crude material 1.05 g (74%) of 6-hydroxyhexyl 2-hexyldecanoate were obtained after precipitation of 1,6-hexanediol in hexane and flash chromatography (hexane:ethylacetate 0-15%).

The organic phase obtained from the previous step after gravitational separation, was pumped through 1/16" Tefzel^{™} tubing (I.D. 1 mm) using an HPLC pump at a flow rate of 1.5 mL min⁻¹ [Line E]. A solution of TEMPO ((2,2,6,6-Tetramethylpiperidin-1-yl)oxyl) in 2-methyltetrahydrofuran (0.50 M) was pumped through 1/16" Tefzel^{™} tubing (I.D. 1 mm) using an HPLC pump at a flow rate of 0.15 mL min⁻¹ [Line F]. Line F was combined with Line E via a T-mixer [Line G] providing a continuous flow of a solution. A solution of both NaOCI and Na₂CO₃ in water (2 M and 0.3 M respectively) was pumped through 1/16" Tefzel^{™} tubing (I.D. 1 mm) using a continuous syringe pump at a flow rate of 1.25 mL min⁻¹ [Line H]. A solution of KBr and NaHCO₃ in water (0.25 M and 0.7 M respectively) was pumped through 1/16" Tefzel^{™} tubing (I.D. 1 mm) using an HPLC pump at a flow rate of 1.5 mL min⁻¹ [Line I]. Line G and Line H were combined in a crossmixer with Line I to provide a continuous biphasic stream which was cooled to room temperature in a 10 mL reactor (Reactor 4, 1/16" Tefzel^{™} tubing (I.D. 1 mm), 2.3 min residence time). The reaction mixture color turned from red (near the cross mixer) to almost colorless (toward the end) through the reactor.

After reaching steady state (2 residence times) the biphasic outlet was collected for 10 minutes. After manual separation of phases, an aliquot of the organic phase was analyzed by quantitative NMR with 1,3,5-trimethoxybenzene as internal standard. Collection over 10 minutes gave 13 mL of organic phase, with 0.57 M (avg. of three experiment) aldehyde concentration, corresponding to 64% yield overall yield over 3 steps.

Organic phase from reactor 4 was dried over Na₂SO₄ and pumped through 1/16" Tefzel^{™} tubing (I.D. 1 mm) using a syringe pump at a flow rate of 0.0777 mL min⁻¹ [Line J]. Tetramethylammonium triacetoxyborohydride in 1-methylpyrrolidin-2-on (0.45 M) was pumped through 1/16" Tefzel^{™} tubing (I.D. 1 mm) using a syringe pump at a flow rate of 0.0984 mL min⁻¹ [Line K]. 4-Aminobutan-1-ol in methanol (0.2 M) was pumped through 1/16" Tefzel^{™} tubing (I.D. 1 mm) using a syringe pump at a flow rate of 0.0738 mL min⁻¹ [Line L]. The lines J, K, L were joined in a crossmixer, which provided a continuous stream that was cooled to room temperature in a 4 mL reactor (Reactor 5, 1/16" Tefzel^{™} tubing (I.D. 1 mm), 16 min residence time). After reaching steady state (two residence times), the outlet was collected for 50 min and quenched with water, hexanes added, basified to pH 9.5 and the phases were separated. The organic phase dried over Na₂SO₄ yielded 1.265 g of crude red oil after solvent removal.

An aliquot of crude (476 mg) was purified on a plug of silica, by sequential washes with hexanes (with 0.1 v% of TFA), hexanes/ethylacetate (4:1, with 0.1 v% of TFA) and pure ethylacetate (with 0.1 v% of TFA). Washing the combined fractions of ethylacetate with Na₂CO₃ and removing the solvent under reduced pressure yielded 121 mg of a slightly yellow oil (yield 57% in last step, based on aminobutanol, 38% based on aldehyde) with an overall yield over the four steps of 25%.

### Example 4: Preparation of a vaccine formulation comprising lipid nanoparticles

Cationic lipid ALC-0315 (0.43 mg) obtained in Example 1, DSPC (0.09 mg), cholesterol (0.2 mg) and ALC-0159 (2-[(polyethylene glycol)-2000]-N,N ditetradecylacetamide, 0.05 mg) were solubilized in ethanol. Lipid nanoparticles (LP) were prepared at a total lipid to mRNA weight ratio of approximately 25:1. The mRNA (30 µg) was diluted to 0.2 mg/mL in citrate buffer, pH 4. Syringe pumps were used to mix the ethanolic lipid solution with the mRNA aqueous solution at a ratio of about 1:5 to 1:3 (vol/vol) with total flow rates above 15 mL/min. The ethanol was then removed and the external buffer replaced with PBS by dialysis. Finally, the lipid nanoparticles were filtered through a 0.2 µπ pore sterile filter. Lipid nanoparticle particle size was approximately 55-95 nm diameter, and in some instances approximately 70-90 nm diameter as determined by quasi-elastic light scattering.

## Claims

1. A continuous flow process for producing a compound of general formula (I) 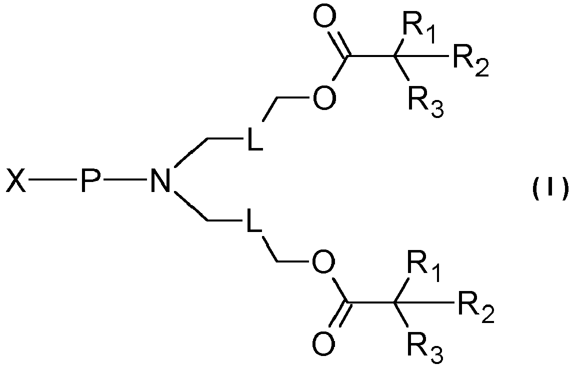 wherein R₁, R₂ and R₃ independently from each other represent -H, C₁₋₂₄ alkyl , or C₂₋₂₄ alkenyl;
L represents C₁₋₁₂ alkylene, C₂₋₁₂ alkenylene, C₂₋₁₂ alkynylene, C₃₋₁₅ cycloalkylene, C₃₋₁₅ cycloalkenylene, or -(CH₂-O-CH₂)₁₋₆-;
P represents C₁₋₂₄ alkylene, C₂₋₂₄ alkenylene, C₂₋₂₄ alkynylene, or -(CH₂)ₘ-(CH₂-O-CH₂)₁₋₆-(CH₂)ₙ-;
X is selected from -H, -OR₄, -CN, -C(O)OR₄, -OC(O)OR₄, and -NR₄R₅;
R₄ and R₅ independently from each other are selected from -H or C₁₋₁₂ alkyl;
wherein m and n are integers independently selected from 0 and 1;
the process comprising the following steps:
A) providing a continuous flow of a solution comprising acid chloride **(II)** and diol compound **(III)**; 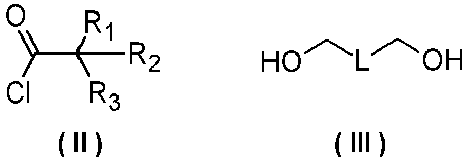
B) continuously producing a compound of formula **(IV)** from the continuous flow of the solution comprising acid chloride **(II)** and diol compound **(III)** in a continuous flow reactor in the absence of a base; 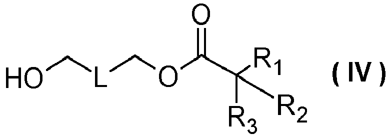
C) combining the continuous flow of a solution containing the compound of formula (**IV**) with a continuous flow of a mixture comprising an oxidizing agent;
D) continuously producing a compound of formula **(V)** from the combined continuous flow obtained in step C) in a continuous flow reactor; 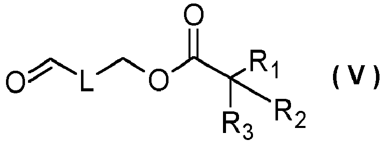
E) combining the continuous flow containing the compound of formula **(V)** obtained in step D) with a continuous flow of a solution comprising a reducing agent and with a continuous flow of a solution comprising an amine compound of formula **(VI)**
X-P-NH₂ **( VI )**
F) continuously producing the compound of formula **(I)** from the combined continuous flow obtained in step E) in a continuous flow reactor.

2. The continuous flow process according to claim 1, wherein the continuous flow of a mixture comprising an oxidizing agent of step C) is obtained by combining i) an aqueous solution comprising the oxidizing agent sodium hypochlorite, a base and an alkali bromide salt, and ii) an organic solution of a piperidin-1-oxyl nitroxyl radical compound and/or a pyrrolidine-1-oxyl nitroxyl radical compound.

3. The continuous flow process according to claim 1 or 2, wherein the continuous flow of a mixture comprising an oxidizing agent of step C) is obtained by combining i) an aqueous solution comprising the oxidizing agent sodium hypochlorite, sodium bicarbonate and potassium bromide, and ii) a solution of (2,2,6,6-tetramethyl-piperidin-1-yl)oxyl in 2-methyl tetrahydrofuran.

4. The continuous flow process according to any one of the claims 1 - 3, wherein following step is performed after step D)
D') purifying compound of formula **(V)** under continuous flow conditions.

5. The continuous flow process according to any one of the claims 1 - 4, further comprising steps B1'), B2') and B3') after step B)
B1') combining the continuous flow of a solution containing the compound of formula (**IV**) with a continuous flow comprising an aqueous base,
B2') continuously performing a neutralization reaction in a continuous flow reactor at a reaction temperature below 23°C, and
B3') purifying compound of formula **(IV)** under continuous flow conditions.

6. The continuous flow process according to any one of the claims 1 - 5, wherein the reducing agent of step E) is a boron compound selected from: NaBH₄, NaBH₄/Celite/LiCl, NaCNBH₃, NaBH(OAc)₃, Me₄NBH(OAc)₃, or borane dimethylsulfide.

7. The continuous flow process according to any one of the claims 1 - 6, wherein the combined continuous flow obtained from step E) comprises at least one C₁₋₅ alcohol and an aprotic solvent.

8. The continuous flow process according to any one of the claims 1 - 7, wherein the combined continuous flow obtained from step E) comprises methanol and 2-methyl tetrahydrofuran.

9. The continuous flow process according to any one of the claims 1 - 8, wherein in step E) the continuous flow containing the compound of formula **(V)** is combined simultaneously with the continuous flow of a solution comprising a reducing agent and with the continuous flow of a solution comprising an amine compound of formula **(VI)** using a cross-shaped mixer.

10. The continuous flow process according to any one of the claims 1 - 9, wherein the continuous flow of a solution comprising acid chloride **(II)** and the diol compound **(III)** of step A) is obtained by combining a continuous flow of a solution comprising the acid chloride **(II)** and a continuous flow of a solution comprising the diol compound **(III)**.

11. The continuous flow process according to claim 10, wherein the continuous flow of a solution comprising the acid chloride **(II)** is obtained from reacting a carboxylic acid of formula **(VII)** with a chlorinating agent, preferably thionyl chloride, oxalyl chloride, bis(trichloromethyl)carbonate or phosgene, in a continuous flow reactor at a reaction temperature between 23°C and 100°C 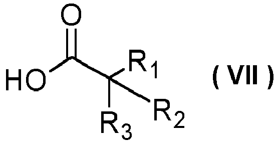 wherein R₁, R₂ and R₃ have the meanings as defined in claim 1.

12. The continuous flow process according to any one of the claims 1 - 11, further comprising step G):
G) isolating the compound of formula **(I)** produced in step F).

13. The continuous flow process according to any one of the claims 1 - 12, further comprising step G*) after step G) or instead of step G):
G*) preparing a vaccine formulation containing the compound of formula **(I)**.

14. The continuous flow process according to any one of the claims 1 - 13, wherein R₃ represents -H and/or wherein X represents -OH.

15. The continuous flow process according to any one of the claims 1 - 14, wherein
| | |
|---|---|
| R₁ | represents -(CH₂)₅-CH₃ ; |
| R₂ | represents -(CH₂)₇-CH₃ ; |
| R₃ | represents -H ; |
| L | represents -(CH₂)₄- ; |
| P | represents -(CH₂)₄- ; and |
| X | represents -OH. |
